# EUROPEAN PATENT APPLICATION

(11) **EP 4 834 360 A1**
(43) Date of publication of application: **04.11.2026**
(21) Application number: 24912860.4
(22) Date of filing: 25.12.2024
(51) Int. Cl.: A61K 38/44, A61P 37/08, C12N 9/02, C12N 15/53

(54) **PROPHYLACTIC AND THERAPEUTIC AGENT FOR DISEASES CAUSED BY TYPE I ALLERGIC REACTION ACCOMPANYING ADMINISTRATION OF ANTICANCER AGENT**

(30) Priority: 26.12.2023 JP 2023219327
(71) Applicant: LTT Bio-Pharma Co., Ltd., Tokyo, 105-0013 (JP); Musashino University, Tokyo 135-8181 (JP)
(72) Inventor: MIZUSHIMA, Tohru, Tokyo 105-0013 (JP); TANJI, Hayato, Tokyo 105-0013 (JP); OGIHARA, Kota, Tokyo 105-0013 (JP); ISHIKAWA, Tomohiro, Tokyo 105-0013 (JP); TSUKIYAMA, Naohiro, Fujisawa-shi, Kanagawa 251-8555 (JP); TANAKA, Ken-ichiro, Nishitokyo-shi, Tokyo 202-8585 (JP)
(74) Representative: Wächtershäuser & Hartz Patentanwaltspartnerschaft mbB
(86) International application number: PCT/JP2024/045804
(87) International publication number: WO 2025/142974

(57) **Abstract**

To provide a prophylactic and therapeutic agent for a disease caused by a type-I allergic reaction induced by administration of a platinum-based drug and/or a taxane-based anticancer agent. The present invention relates to a pharmaceutical composition comprising a lecithinized superoxide dismutase, for treating or preventing a disease caused by a type-I allergic reaction induced by administration of a platinum-based drug and/or a taxane-based anticancer agent.

## Description

### Technical Field

The present invention relates to a prophylactic and therapeutic agent for a disease caused by a type-I allergic reaction induced by administration of an anticancer agent.

### Background Art

With development of many anticancer agents, the effect of drug therapies on cancer has been dramatically improved. However, adverse drug reactions caused by anticancer agents have also become evident. In particular, the incidence of diseases caused by type-I allergic reactions induced by administration of platinum-based drugs, exemplified by cisplatin, carboplatin, and oxaliplatin, and taxane-based anticancer agents is observed in from 10 to 20% of patients. Symptoms thereof include fever, nausea, chills, headache, rash, eruption, itching, swelling, tachycardia, hives, flushing, throat tightness, coughing, difficulty breathing, and the like. In severe cases, wheezing, loss of consciousness, hypotension, and anaphylaxis accompanied by respiratory problems may occur.

If type-I allergic symptoms occur following the administration of platinum-based drugs and/or taxane-based anticancer agents, further administration of these agents is generally avoided. Therefore, effective treatment can be discontinued due to such adverse reactions, and the therapeutic benefits of these anticancer agents may not be achieved.

When intensifying premedication, it is common practice to add 5 mg of d-chlorpheniramine maleate injection and 20 mg of famotidine or 50 mg of ranitidine, increase the dose of dexamethasone, and slow the infusion rate of the target drug (e.g., extending the administration time to more than twice the usual duration).

### Citations

### Patent Literature

Patent Literature 1: JP-A-2006-169128
Patent Literature 2: JP-A-2008-75706
Patent Literature 3: JP-A-2010-215527
Patent Literature 4: JP-A-2017-73255
Patent Literature 5: JP-A-2001-151695
Patent Literature 6: WO 2022/244757
Patent Literature 7: JP-A-2001-64199
Patent Literature 8: JP-A-hei-6-199895
Patent Literature 9: WO 2006/040980

### Summary of Invention

### Technical Problem

As described above, if type-I allergic symptoms are observed following the administration of platinum-based drugs and/or taxane-based anticancer agents, the subsequent administration of these drugs is generally not performed, and therefore, effective treatment cannot be continued in many cases. Thus, there is a demand for the development of drugs which can suppress or prevent these side effects.

Therefore, the present invention aims to provide a prophylactic and therapeutic agent for a disease caused by a type-I allergic reaction induced by administration of a platinum-based drug and/or a taxane-based anticancer agent.

### Solution to Problem

Accordingly, the present inventors focused on a lecithinized superoxide dismutase (PC-SOD) and elucidated a pharmacological action thereof from various points of view. As a result, the present inventors found that PC-SOD strongly suppresses the onset of diseases caused by type-I allergic reactions induced by administration of platinum-based drugs and/or taxane-based anticancer agents and that PC-SOD exhibits high level of safety, and thus completed the present invention.

Specifically, the present invention provides the following [1] to [12]:
[1] A pharmaceutical composition comprising a lecithinized superoxide dismutase, for treating or preventing a disease caused by a type-I allergic reaction induced by administration of a platinum-based drug and/or a taxane-based anticancer agent, the pharmaceutical composition.
[2] The pharmaceutical composition according to [1], wherein the platinum-based drug and/or the taxane-based anticancer agent is one or more selected from the group consisting of cisplatin, carboplatin, oxaliplatin, nedaplatin, docetaxel, and paclitaxel.
[3] The pharmaceutical composition according to [1] or [2], wherein the disease caused by a type-I allergic reaction is one or more diseases selected from the group consisting of fever, nausea, chills, headache, rash, eruption, itching, swelling, tachycardia, hives, flushing, throat tightness, coughing, difficulty breathing, wheezing, loss of consciousness, hypotension, and anaphylaxis accompanied by respiratory problems.
[4] The pharmaceutical composition according to any one of [1] to [3], which is administered in combination with a platinum-based drug and/or a taxane-based anticancer agent.
[5] The pharmaceutical composition according to any one of [1] to [4], which is administered before administration of a platinum-based drug and/or a taxane-based anticancer agent.
[6] The pharmaceutical composition according to any one of [1] to [5], wherein the lecithinized superoxide dismutase is lyophilized.
[7] The pharmaceutical composition according to any one of [1] to [6], comprising a lecithinized superoxide dismutase,
   the lecithinized superoxide dismutase comprising a lecithinized superoxide dismutase in which at least one amino group in a human-derived superoxide dismutase subunit coordinated with copper and/or zinc and having a mercapto group of cysteine at position 111 replaced by a hydroxyethylthio group is modified with a lecithin residue of the following formula (I): 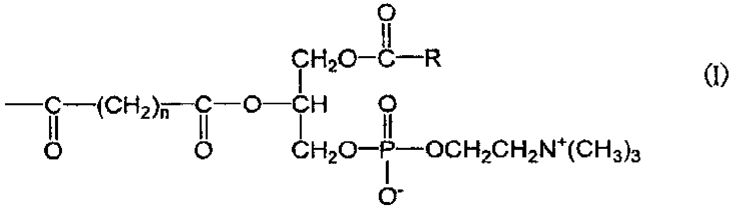 [wherein R represents an alkyl group with 8 to 30 carbon atoms, and n is an integer of from 2 to 10] directly or via a linker, and
   the lecithinized superoxide dismutase comprises a lecithinized superoxide dismutase (A), as a principal component, constituted by lecithinized superoxide dismutase subunits each of which independently has m number of amino groups [m is an integer of from 1 to 4, and the mean value of m in lecithinized superoxide dismutase subunits is from 1.5 to 2.4] substituted with the lecithin residue, wherein
   the lecithinized superoxide dismutase (A) is constituted by the following lecithinized superoxide dismutase subunits:
      from 25 to 40 mol% lecithinized superoxide dismutase subunit (a1) with m = 1,
      from 35 to 50 mol% lecithinized superoxide dismutase subunit (a2) with m = 2,
      from 10 to 20 mol% lecithinized superoxide dismutase subunit (a3) with m = 3, and
      from 5 to 15 mol% lecithinized superoxide dismutase subunit (a4) with m = 4.
[8] The pharmaceutical composition according to any one of [1] to [7], wherein PC molecules bind to each SOD molecule with the following binding rates in the lecithinized superoxide dismutase:
   (1) the PC binding rate at the binding sites of K3, N terminus, and T2 is from 1% to 10% as the sum of these amino acid residues;
   (2) the PC binding rate at the binding site of K9 is from 10% to 30%;
   (3) the PC binding rate at the binding site of K23 is from 50% to 75%;
   (4) the PC binding rate at the binding site of K36 is from 30% to 55%;
   (5) the PC binding rate at the binding site of K70 or K75 is from 10% to 80% as the sum of these amino acid residues;
   (6) the PC binding rate at the binding site of K122 or K128 is from 10% to 45% as the sum of these amino acid residues;
   (7) the PC binding rate at binding site of K136 is from 10% to 60%; and
   on average 1 to 8 PC molecules are bound in a PC-SOD molecule as the sum of the (1) to (7).
[9] The pharmaceutical composition according to any one of [1] to [8], wherein a superoxide dismutase in the lecithinized superoxide dismutase has an amino acid sequence set forth in SEQ ID NO: 1.
[10] Use of a lecithinized superoxide dismutase to manufacture a pharmaceutical composition for treating or preventing a disease caused by a type-I allergic reaction induced by administration of a platinum-based drug and/or a taxane-based anticancer agent.
[11] A lecithinized superoxide dismutase for treating or preventing a disease caused by a type-I allergic reaction induced by administration of a platinum-based drug and/or a taxane-based anticancer agent.
[12] A method for treating or preventing a disease caused by a type-I allergic reaction induced by administration of a platinum-based drug and/or a taxane-based anticancer agent, comprising administration of a lecithinized superoxide dismutase.

### Advantageous Effects of Invention

The PC-SOD used in the present invention is useful for suppressing and preventing a disease caused by a type-I allergic reaction induced by administration of a platinum-based drug and/or a taxane-based anticancer agent. Such diseases include one or more selected from the group consisting of fever, nausea, chills, headache, rash, eruption, itching, swelling, tachycardia, hives, flushing, throat tightness, coughing, difficulty breathing, wheezing, loss of consciousness, hypotension, and anaphylaxis accompanied by respiratory problems. In addition, PC-SOD exhibits a high level of safety.

### Brief Description of Drawings

Fig. 1 shows that the incidence of oxaliplatin allergy was suppressed by combining PC-SOD with mFOLFOX6 therapy as compared with placebo (p = 0.0274).
Fig. 2 shows changes in IgE antibody levels due to oxaliplatin administration.
Fig. 3 shows that PC-SOD has an inhibitory effect on the increase in IgE levels caused by BSA-OXA, whereas SOD does not exhibit such an inhibitory effect.

### [Modes for Carrying Out the Invention]

An embodiment of the present invention is a pharmaceutical composition for treating or preventing a disease caused by a type-I allergic reaction induced by administration of a platinum-based drug and/or a taxane-based anticancer agent, the pharmaceutical composition comprising a lecithinized superoxide dismutase.

An active ingredient of the pharmaceutical composition of the present invention is a lecithinized superoxide dismutase (PC-SOD).

In the present specification, the terms "lecithinized superoxide dismutase" and "PC-SOD" are synonymous and can be used interchangeably.

In the present specification, the terms "superoxide dismutase" and "SOD" are synonymous and can be used interchangeably.

In the present specification, the terms "phosphatidylcholine," "PC," and "lecithin" are synonymous, can be used interchangeably, and can be used for both an aggregate and individual molecules.

In the present specification, the term "PC-SOD" means an aggregate of PC-SOD molecules.

Here, the number of PC molecules binding to an individual PC-SOD molecule constituting "PC-SOD" and binding sites thereof may be the same or different between PC-SOD molecules. Therefore, the number of PC molecules binding to "PC-SOD" can be represented by the average number of binding PC molecules. In "PC-SOD", the PC binding site can be identified by an amino acid residue in the SOD, and the PC binding rate can be represented by the percent of PC molecules binding to amino acid residues in an SOD.

In the present specification, the term "PC-SOD subunit" means an aggregate of PC-SOD molecule subunits.

In the present specification, the term "SOD" means an aggregate of SOD molecules.

In the present specification, the term "SOD subunit" means an aggregate of SOD molecule subunits.

In the present specification, the term "PC-SOD molecule" means a dimeric molecule consisting of PC-SOD molecule subunits, and the term "PC-SOD molecule subunit" means a monomer constituting a dimeric PC-SOD molecule. Here, the number and the binding site of PC molecules binding a PC-SOD molecule subunit constituting each PC-SOD molecule dimer may be the same or different between PC-SOD molecule subunits constituting the dimer.

In the present specification, the term "SOD molecule" means a molecule obtained by dimerizing SOD molecule subunits, and the term "SOD molecule subunit" means a monomer constituting a dimeric SOD molecule.

PC-SOD is a molecule obtained by modifying SOD with lecithin. The applicant of the present invention has reported so far that PC-SOD is effective for burn (Patent Literature 1), interstitial pneumonia (Patent Literature 2), chronic obstructive pulmonary disease syndrome (Patent Literature 3), acute respiratory distress syndrome (Patent Literature 4), type III allergy (Patent Literature 5), neuropathy induced by administration of an anticancer agent (Patent Literature 6), and the like. However, the action of PC-SOD on a disease caused by a type-I allergic reaction induced by administration of a platinum-based drug and/or a taxane-based anticancer agent is unknown.

PC-SOD is a molecule obtained by modifying SOD with lecithin and it is preferably a molecule obtained by modifying one or more amino acid residues of SOD, preferably an amino acid residue containing an amino group or an N terminal amino acid residue, more preferably a lysine residue with phosphatidylcholine (PC) directly or via a linker.

As SOD in PC-SOD, a human SOD (SEQ ID NO: 1) is preferred, and a human SOD having copper and zinc at the active center (human Cu/Zn SOD) is particularly preferred. Examples of this SOD include a natural human Cu/Zn SOD manufactured from human tissue, a human Cu/Zn SOD manufactured using a genetic engineering technique, a recombinant human Cu/Zn SOD having an amino acid sequence substantially identical to that of a natural human Cu/Zn SOD, and an SOD obtained by chemically modifying or altering some amino acids in the amino acid sequences of these human Cu/Zn SOD molecules, and any of these human Cu/Zn SOD molecules may be used. The SOD in PC-SOD is preferably a human SOD 1, more preferably a human SOD 1 in which a mercapto group of cysteine at position 111 is protected, further more preferably a human SOD 1 in which a mercapto group of cysteine at position 111 is replaced by a hydroxyethylthio group.

In PC-SOD, PC binds directly or via a linker to one or more free amino groups or hydroxy groups (preferably amino groups) among amino acid residues constituting SOD. Examples of an amino acid residue to which PC binds include Ala at the N terminus of an SOD molecule and Lys, Gln, Asn, Arg, Ser, Thr, and Tyr, preferably Ala at the N terminus and Lys and Thr, more preferably Ala at the N terminus and Lys, further more preferably Lys.

Preferably 1 to 12 PC molecules, more preferably 1 to 8 PC molecules, further more preferably 1 to 4 PC molecules, even more preferably 2 to 3 PC molecules, further preferably 2 PC molecules are independently bound to each SOD molecule subunit.

Therefore, preferably 2 to 24 PC molecules, more preferably 2 to 16 PC molecules, further more preferably 2 to 8 PC molecules, even more preferably 4 to 6 PC molecules, further more preferably 4 PC molecules are bound to a PC-SOD molecule.

For PC-SOD, the number of PC molecules binding to a SOD molecule can be expressed with the average number of PC molecules binding to an aggregate of SOD molecules. Therefore, in PC-SOD, 2 to 24 PC molecules, preferably 2 to 16 PC molecules, more preferably 2 to 8 PC molecules, further more preferably 4 to 6 PC molecules, most preferably 4 PC molecules are bound to an SOD molecule on average.

PC may bind to an amino acid residue constituting SOD directly, but preferably via a linker.

As a linker, dicarboxylic acid is preferred, - C(=O)(CH₂)ₙC(=O)- is particularly preferred (here, n is an integer of 2 or greater, preferably from 2 to 10, more preferably from 2 to 6, further more preferably from 2 to 4, particularly preferably 3). It should be noted that this linker links a hydroxy group of a glyceryl group in PC and an amino group or a hydroxy group (preferably amino group) of an amino acid residue.

A fatty acid residue in PC has preferably 8 to 31 carbon atoms, more preferably 10 to 28 carbon atoms, further more preferably 14 to 22 carbon atoms, particularly preferably 16 carbon atoms (derived from palmitic acid).

PC-SOD can be manufactured by, for example, the methods described in Patent Literatures 7, 8, and 9, preferably the method described in Patent Literature 9.

A particularly preferred example of PC-SOD is a lecithinized superoxide dismutase,
the lecithinized superoxide dismutase comprising a lecithinized superoxide dismutase in which at least one amino group in a human-derived superoxide dismutase subunit coordinated with copper and/or zinc and having a mercapto group in cysteine at position 111 replaced by a hydroxyethylthio group is modified with a lecithin residue of the following formula (I): 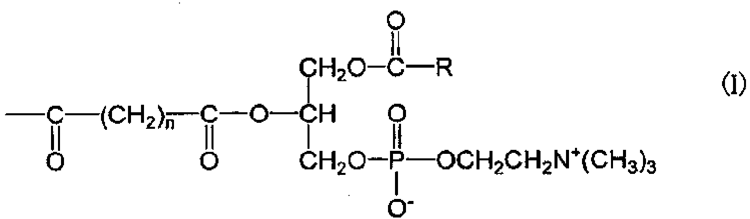 [wherein R represents an alkyl group with 8 to 30 carbon atoms, and n is an integer of from 2 to 10] directly or via a linker, wherein
the lecithinized superoxide dismutase comprises a lecithinized superoxide dismutase (A), as a principal component, constituted by lecithinized superoxide dismutase subunits each of which independently has m of amino groups [m is an integer of from 1 to 4, and the mean value of m in lecithinized superoxide dismutase subunits is from 1.5 to 2.4] substituted with the lecithin residue, wherein
the lecithinized superoxide dismutase (A) is constituted by the following lecithinized superoxide dismutase subunits:
   from 25 to 40 mol% lecithinized superoxide dismutase subunit (a1) with m = 1,
   from 35 to 50 mol% lecithinized superoxide dismutase subunit (a2) with m = 2,
   from 10 to 20 mol% lecithinized superoxide dismutase subunit (a3) with m = 3, and
   from 5 to 15 mol% lecithinized superoxide dismutase subunit (a4) with m = 4.

In the PC-SOD of the present invention, the binding sites and the binding rates of PC binding to SOD are preferably as described below. It should be noted that the number after a one-letter amino acid code represents the number of residues in an SOD molecule from the N terminus.
(1) When the binding site is K3, the N terminus, or T2, the PC binding rate is preferably from 1% to 10%, more preferably from 2% to 8%, further more preferably 5% as the sum of these amino acid residues.
(2) When the binding site is K9, the PC binding rate is preferably from 10% to 30%, more preferably from 15% to 26%, further more preferably 23%.
(3) When the binding site is K23, the PC binding rate is preferably from 50% to 75%, more preferably from 55% to 70%, further more preferably 63%.
(4) When the binding site is K36, the PC binding rate is preferably from 30% to 55%, more preferably from 35% to 50%, further more preferably 43%.
(5) When the binding site is K70 or K75, the PC binding rate is preferably from 10% to 80%, more preferably from 30% to 65%, further more preferably 53% as the sum of these amino acid residues.
(6) When the binding site is K122 or K128, the PC binding rate is preferably from 10% to 45%, more preferably from 20% to 35%, further more preferably 27% as the sum of these amino acid residues.
(7) When the binding site is K136, the PC binding rate is preferably from 10% to 60%, more preferably from 20% to 45%, further more preferably 33%.

As the sum of the above-mentioned (1) to (7), preferably 1 to 8 PC molecules, preferably 2 to 6 PC molecules, more preferably 3 to 5 PC molecules, most preferably 4 PC molecules bind to PC-SOD on average.

As described in the examples described later, the PC-SOD has a therapeutic or prophylactic effect on a disease caused by a type-I allergic reaction induced by administration of a platinum-based drug and/or a taxane-based anticancer agent.

Furthermore, the present inventors conducted studies to clarify the mechanism of action of the administration of PC-SOD for treating or preventing a disease caused by a type-I allergic reaction induced by administration of a platinum-based drug and/or a taxane-based anticancer agent. As a result, they found that (1) PC-SOD suppresses the increase in IgE antibodies induced by administration of a platinum-based drug and/or a taxane-based anticancer agent, and that (2) PC-SOD also suppresses allergic reactions, such as increased vascular permeability, induced by administration of a platinum-based drug and/or a taxane-based anticancer agent. It was also confirmed that SOD does not suppress the increase in IgE antibodies induced by administration of a platinum-based drug and/or a taxane-based anticancer agent.

The disease caused by a type-I allergic reaction induced by administration of a platinum-based drug and/or a taxane-based anticancer agent is, for example, one or more diseases selected from the group consisting of fever, nausea, chills, headache, rash, eruption, itching, swelling, tachycardia, hives, flushing, throat tightness, coughing, difficulty breathing, wheezing, loss of consciousness, hypotension, and anaphylaxis accompanied by respiratory problems.

Furthermore, the platinum-based drug is, for example, one or more selected from the group consisting of cisplatin, carboplatin, oxaliplatin, and nedaplatin. The taxane-based anticancer agent is, for example, docetaxel or paclitaxel.

The pharmaceutical composition of the present invention is preferably administered in cancer chemotherapy involving administration of a platinum-based drug and/or a taxane-based anticancer agent, such as FOLFOX therapy, XELOX therapy (CapeOX therapy), or TC therapy.

FOLFOX is a cancer chemotherapy in which folinic acid, fluorouracil, and oxaliplatin are used in combination. FOLFOX has variations of modified methods depending on the drug dose differences, including FOLFOX1 to FOLFOX7, and mFOLFOX6, and FOLFOX4, FOLFOX6, and mFOLFOX6 are preferred. mFOLFOX6 is particularly preferred in Japan.

XELOX therapy (CapeOX therapy) is a cancer chemotherapy in which Xeloda and oxaliplatin (capecitabine and oxaliplatin) are used in combination.

TC therapy is a cancer chemotherapy in which paclitaxel and carboplatin are used in combination.

The pharmaceutical composition of the present invention is preferably administered to a cancer patient suffering from a cancer which can be treated with a platinum-based drug and/or a taxane-based anticancer agent, for example, one or more cancers selected from the group consisting of ovarian cancer, non-small cell lung cancer, breast cancer, endometrial cancer, head and neck cancer, esophageal cancer, leukemia, malignant lymphoma, pediatric tumor, multiple myeloma, malignant astrocytoma, neuroglioma, chorionic disease, germ cell tumor, lung cancer, testicular tumor, bladder cancer, renal pelvic tumor, urethral tumor, prostate cancer, uterine cervical cancer, neuroblastic tumor, small-cell lung cancer, bone sarcoma, malignant pleural mesothelioma, malignant bone tumor, kidney cancer, penis cancer, each bone and soft tissue tumor, liver cancer, thyroid gland cancer, retroperitoneal tumor, bone metastasis, testicular cancer, gallbladder cancer, biliary tract cancer, biliary duct cancer, adrenal cancer, neuroblastoma, hepatoblastoma, primary hepatic malignant tumor, medulloblastoma, stomach cancer, pancreas cancer, urothelial cancer, extragonadal tumor, Langerhans cell histiocytosis, mantle cell lymphoma, lymphoplasmacytic lymphoma, small intestine cancer, colon cancer, rectal cancer, and large intestine cancer, more preferably a cancer patient suffering from one or more cancers selected from the group consisting of stomach cancer, pancreas cancer, small intestine cancer, colon cancer, rectal cancer, and large intestine cancer, further more preferably a cancer patient suffering from one or more cancers selected from the group consisting of colon cancer, rectal cancer, and large intestine cancer.

In the present invention, a platinum-based drug and/or a taxane-based anticancer agent can also be administered as a postoperative adjuvant chemotherapy to a patient from whom cancer was removed or to a patient to prevent recurrence. Specifically, the platinum-based drug and/or the taxane-based anticancer agent is administered preferably to a patient who has a history of one or more cancers selected from the group consisting of ovarian cancer, non-small cell lung cancer, breast cancer, endometrial cancer, head and neck cancer, esophageal cancer, leukemia, malignant lymphoma, pediatric tumor, multiple myeloma, malignant astrocytoma, neuroglioma, chorionic disease, germ cell tumor, lung cancer, testicular tumor, bladder cancer, renal pelvic tumor, urethral tumor, prostate cancer, uterine cervical cancer, neuroblastic tumor, small-cell lung cancer, bone sarcoma, malignant pleural mesothelioma, malignant bone tumor, kidney cancer, penis cancer, each bone and soft tissue tumor, liver cancer, thyroid gland cancer, retroperitoneal tumor, bone metastasis, testicular cancer, gallbladder cancer, biliary tract cancer, biliary duct cancer, adrenal cancer, neuroblastoma, hepatoblastoma, primary hepatic malignant tumor, medulloblastoma, stomach cancer, pancreas cancer, urothelial cancer, extragonadal tumor, Langerhans cell histiocytosis, mantle cell lymphoma, lymphoplasmacytic lymphoma, small intestine cancer, colon cancer, rectal cancer, and large intestine cancer and from whom the cancer is removed, more preferably a patient who has a history of one or more cancers selected from the group consisting of stomach cancer, pancreas cancer, small intestine cancer, colon cancer, rectal cancer, and large intestine cancer and from whom the cancer or cancers were removed, further more preferably a patient who has a history of one or more cancers selected from the group consisting of colon cancer, rectal cancer, and large intestine cancer and from whom the cancer or cancers were removed.

The pharmaceutical composition of the present invention is not limited as long as it comprises PC-SOD, and the pharmaceutical composition is preferably formulated in various dosage forms by mixing pharmacologically acceptable carriers with PC-SOD.

Such a pharmaceutical composition may be administered by injection, preferably intravenous injection, local injection, or drip infusion.

For injection, water, a dissolving aid, an aqueous xylitol solution (preferably 5 w/w%, 10 w/w%, or 20 w/w%, more preferably 5 w/w%), an aqueous glucose solution (preferably 5 w/w%, 10 w/w%, or 20 w/w%, more preferably 5 w/w%), or the like is used. An injection is preferably an intravenous injection, a local injection, a subcutaneous injection, and an intramuscular injection. More preferred is an intravenous injection, a local injection, or a drip infusion.

Examples of dosage forms of the pharmaceutical composition of the present invention include forms of a lyophilized formulation and a powder filler, and a lyophilized formulation is preferred. To prepare a lyophilized formulation, a stabilizer such as sucrose is preferably mixed. The formulation is prepared by lyophilizing an aqueous solution containing PC-SOD and sucrose by a method known in this technical field. The weight ratio of PC-SOD and sucrose is preferably from 1:1 to 1:5, more preferably from 1:1 to 1:2. For 40 mg of PC-SOD, from 40 to 200 mg of sucrose is preferred, from 40 to 80 mg is more preferred, and 67 mg is further more preferred.

The content of PC-SOD in the pharmaceutical composition of the present invention is not particularly limited, but is usually from 1 to 100 mass%, preferably from 10 to 80 mass%, more preferably from 25 to 50 mass% to the total mass of a composition.

Doses of the pharmaceutical composition of the present invention vary depending on symptoms, age, and the like of a patient to whom the anticancer agent is administered, and from 20 to 160 mg per day of the PC-SOD for adults is preferred, from 20 to 80 mg is more preferred, and from 40 to 80 mg is further more preferred. The dose can be divided into one to four times per week, one to four times per two weeks, or one to four times per three weeks, and each dosing cycle can be repeated from once to 20 times. Preferably, the pharmaceutical composition of the present invention can be administered in accordance with the FOLFOX, XELOX or TC dosing regimen. With the FOLFOX dosing regimen, the pharmaceutical composition of the present invention is preferably administered once every two weeks which is repeated 12 cycles. With the XELOX dosing regimen, the pharmaceutical composition of the present invention is preferably administered once every three weeks which is repeated eight cycles. With the TC dosing regimen, the pharmaceutical composition of the present invention is administered once every three weeks which is repeated six cycles.

The pharmaceutical composition of the present invention is preferably administered in combination with a platinum-based drug and/or a taxane-based anticancer agent, preferably administered before or after administration of a platinum-based drug and/or a taxane-based anticancer agent, more preferably administered before administration of a platinum-based drug and/or a taxane-based anticancer agent. More specifically, it is more preferable that the pharmaceutical composition of the present invention is administered within 3 hours, more preferably within 2 hours, further more preferably within 1 hour, before administration of a platinum-based drug and/or a taxane-based anticancer agent. In this case, the pharmaceutical composition of the present invention is preferably administered by intravenous administration, more preferably by intravenous drip infusion.

Another preferred embodiment of the present invention is use of PC-SOD to manufacture a pharmaceutical composition for treating or preventing a disease caused by a type-I allergic reaction induced by administration of a platinum-based drug and/or a taxane-based anticancer agent.

Still another preferred embodiment of the present invention is PC-SOD for treating or preventing a disease caused by a type-I allergic reaction induced by administration of a platinum-based drug and/or a taxane-based anticancer agent.

Still another preferred embodiment of the present invention is a method for treating or preventing a disease caused by a type-I allergic reaction induced by administration of a platinum-based drug and/or a taxane-based anticancer agent, comprising administration of PC-SOD.

Preferred embodiments of the pharmaceutical composition and PC-SOD in these other embodiments of the present invention are the same as in the case of the pharmaceutical composition of the present invention described above.

### Examples

The present invention is described in detail below with reference to examples, but the scope of the present invention is not limited to these examples.

The PC-SOD used in these examples was manufactured in accordance with the method described in Patent Literature 9.

### Example 1

### [Method]

A phase II, placebo-controlled, double-blind comparative study of PC-SOD was conducted on large intestine cancer patients undergoing mFOLFOX6 therapy as postoperative adjuvant chemotherapy (PC-SOD group: 43 cases, placebo group: 47 cases). Twelve cycles of mFOLFOX6 therapy (85 mg/m² of oxaliplatin and 200 mg/m² of levofolinate were administered intravenously over 2 hours, followed by rapid intravenous injection of 400 mg/m² of fluorouracil, and followed by continuous intravenous injection of 2400 mg/m² of fluorouracil for 46 hours; this cycle was performed every two weeks) were conducted. Before oxaliplatin administration in each mFOLFOX6 therapy, a test drug (80 mg of PC-SOD or placebo) was administered intravenously. Efficacy was assessed by defining oxaliplatin-induced allergic reactions as an event, and comparing the time to the first event between treatment groups. One cycle of test-drug administration was treated as a unit of time, and comparisons were performed using the log-rank test.

### [Results]

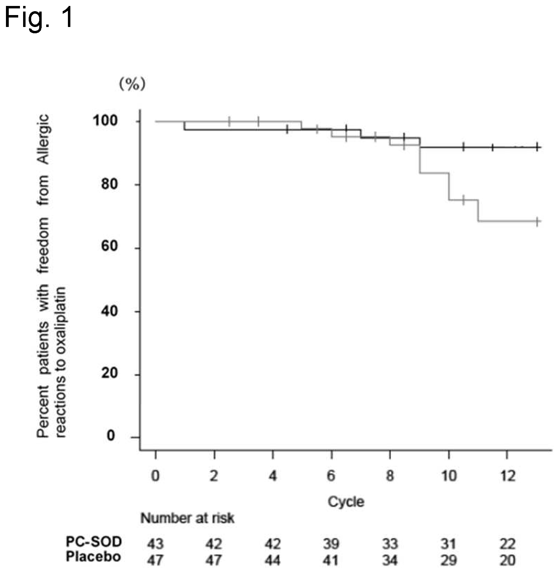

As compared with placebo, the combined use of PC-SOD with mFOLFOX6 therapy suppressed the incidence of oxaliplatin allergy (Fig. 1, p = 0.0274).

### Test Example 1 (Action of oxaliplatin administration on the increase in blood IgE antibodies)

### [Method]

(1) Preparation of oxaliplatin-induced allergy model mice:
   Six to eight-week-old ICR male mice (from 30 to 40 g) were used, and oxaliplatin (hereinafter may be abbreviated as OXA) was administered into the peritoneal cavity of mice at a dose of 12.5 mg/kg. The administration was carried out twice in total, on Days 1 and 11. A solvent for OXA, i.e., sterile saline (Otsuka Pharmaceutical Co., Ltd.), was similarly administered to a control group. Oxaliplatin Intravenous Solution 100 mg (Nipro Corporation) was used as OXA.
(2) Experiment to assess the allergy prophylactic effect of the test drug:
   Laboratory animals were divided into three groups: a control group, an OXA administration group, and a group receiving administration of OXA and PC-SOD 1 mg/kg (OXA + PC-SOD administration group). OXA was administered twice in total, on Days 1 and 11, and PC-SOD was administered daily via caudal vein injection for 5 days starting from Day 0 (Days 0, 1, 2, 3, and 4). A solvent for PC-SOD, i.e., sterile saline (Otsuka Pharmaceutical Co., Ltd.) diluted to half concentration with Milli Q water, was similarly administered to the control group and the OXA administration group.
(3) Measurement of IgE
   Blood was collected from the mice on Days 4, 11, and 21, and IgE levels in the blood were measured using a Mouse IgE ELISA Kit (Bethyl Laboratories, Inc.).

### [Results and interpretation]

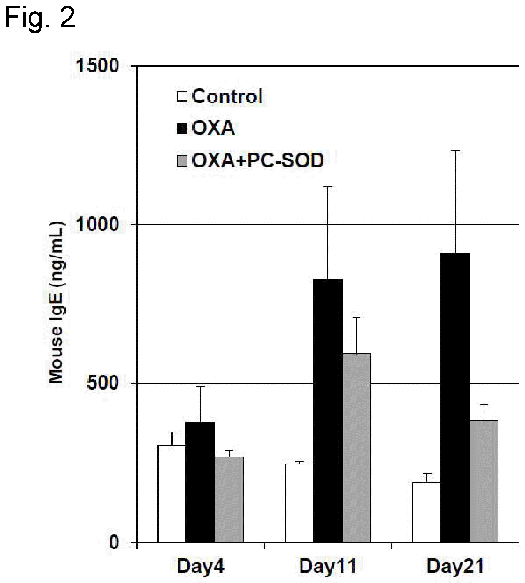

Fig. 2 shows the results of IgE measurements. In the OXA administration group, IgE levels in the blood were increased as compared with the control group. In contrast, in the OXA + PC-SOD administration group, PC-SOD suppressed the increase in blood IgE levels caused by OXA.

From the above results, it was confirmed that PC-SOD had an inhibitory effect on the increase in IgE levels caused by OXA.

### Test Example 2 (Action of bovine serum albumin-oxaliplatin complex administration on the increase in IgE antibodies)

### [Method]

(1) Production of bovine serum albumin-oxaliplatin complex:
   Bovine serum albumin (Sigma-Aldrich Co., Ltd.) and oxaliplatin (Tokyo Chemical Industry Co., Ltd.) were dissolved in D-PBS (FUJIFILM Wako Pure Chemical Corporation) to prepare 20% BSA and 5% OXA solutions. The 20% BSA and 5% OXA solutions were mixed to final concentrations of 5% BSA and 0.14% OXA, respectively, and the mixture was left to stand at 37°C for 72 hours.
(2) Adjustment of bovine serum albumin-oxaliplatin complex:
   The BSA-OXA solution was dialyzed against an adequate volume of D-PBS and subsequently concentrated by ultrafiltration.
(3) Preparation of BSA-OXA-induced allergy model mice:
   Six- to eight-week-old male ICR mice (30-40 g) were used. BSA-OXA was administered into the peritoneal cavity of mice at a dose corresponding to 12.5 mg/kg of OXA. The administration was carried out twice in total, on Days 1 and 11. A 1.87% BSA solution was administered in the same manner to a control group.
(4) Experiment to assess the allergy prophylactic effect of the test drug:
   Laboratory animals were divided into four groups: a control group, a BSA-OXA administration group, a group receiving administration of BSA-OXA and PC-SOD 1 mg/kg (BSA-OXA + PC-SOD administration group), and a group receiving administration of BSA-OXA and SOD 1 mg/kg (BSA-OXA + SOD administration group). OXA was administered twice in total, on Days 1 and 11, and PC-SOD or SOD was administered daily via caudal vein injection for 5 days starting from Day 0 (Days 0, 1, 2, 3, and 4). A vehicle for PC-SOD or SOD, i.e., sterile saline (Otsuka Pharmaceutical Co., Ltd.) diluted to half concentration with Milli Q water, was similarly administered to the control group and the BSA-OXA administration group.
(5) Measurement of IgE
   Blood samples were collected from mice on Day 11, and blood IgE levels were measured using a Mouse IgE ELISA Kit (Bethyl Laboratories, Inc.).

### [Results and interpretation]

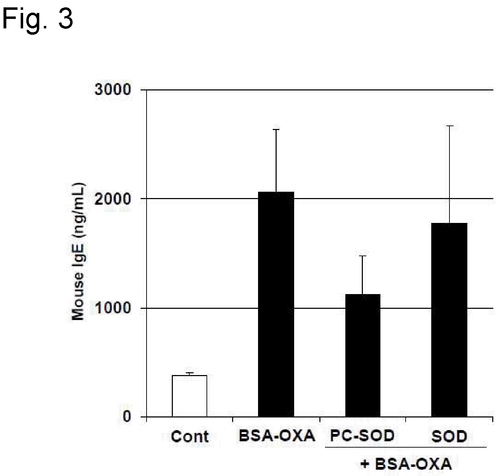

Fig. 3 shows the results of IgE measurements. In the BSA-OXA administration group, blood IgE levels were elevated compared with the control group. In contrast, in the BSA-OXA + PC-SOD administration group, PC-SOD suppressed the BSA-OXA-induced increase in blood IgE levels. Meanwhile, in the BSA-OXA + SOD group, the increase in blood IgE levels caused by BSA-OXA was similar to that in the BSA-OXA administration group.

These results demonstrate that PC-SOD inhibits the increase in IgE levels induced by BSA-OXA, whereas SOD does not exhibit such an inhibitory effect.

### Test Example 3 (Passive cutaneous anaphylaxis (PCA) reaction test)

### [Method]

### (1) Sensitization treatment

Five- to six-week-old male Hartley guinea pigs (Kwl: Hartley) weighing 250 g or more were used as experimental animals. OXA was administered subcutaneously to the guinea pigs at a dose of 10 mg/kg together with Freund's Complete Adjuvant (FCA) as an adjuvant. The administration was carried out three times in total at 14-day intervals (Days 0, 14, and 28). A solvent for OXA, i.e., a 5% glucose solution, was administered in the same manner to a control group.

### (2) Blood collection

To obtain serum for the Homo-PCA reaction, OXA was administered to the auricular vein of guinea pigs at a dose of 12 mg/kg 42 days after sensitization. Two days later (44 days after sensitization), whole blood was collected from the abdominal aorta under isoflurane anesthesia. The blood was centrifuged at about 1,600 × g for 20 minutes to obtain serum, and the resulting serum was used as serum for the Homo-PCA reaction.

### (3) Homo-PCA reaction

One day before the PCA reaction, the hair on the backs of all animals was clipped with an electric clipper (Daito Electric Machine Industry Co., Ltd.) and, if necessary, shaved with an electric razor (Braun).
1) On the day of the PCA reaction, the serum prepared for the Homo-PCA reaction was diluted two-fold with physiological saline.
2) A 0.1 mL aliquot of the serum diluted in 1) was administered intradermally at one site (site 1) on the backs of all animals in each group.
3) After a 3-hour sensitization period, 3 mg/kg of PC-SOD or its vehicle, a 5% xylitol solution (Fuso Pharmaceutical Industries, Ltd.), was administered in the same manner.
4) About one hour later, a challenge agent (OXA, 12 mg/mL) containing Evans blue was injected into the auricular vein.
5) 30 minutes after challenge, the abdominal aorta was severed under deep isoflurane anesthesia to euthanize the animals by exsanguination, after which the dorsal skin was removed.
6) All the excised skin samples were photographed using a digital camera.

### [Results and interpretation]

Table 1 shows the results of the Homo-PCA reaction. In the control group, pigment leakage was observed in 50% of the individuals, whereas that in the PC-SOD administration group was 25%, which showed that pigment leakage was suppressed in the PC-SOD administration group as compared with the control group. The above results shows that PC-SOD has a prophylactic effect on increased vascular permeability, which is a type of allergic reaction caused by OXA administration.

**[Table 1]**

| Group | PCA reaction (incidence) |
|---|---|
| Solvent administration group | 50% |
| PC-SOD administration group | 25% |

## Claims

1. A pharmaceutical composition comprising a lecithinized superoxide dismutase, for treating or preventing a disease caused by a type-I allergic reaction induced by administration of a platinum-based drug and/or a taxane-based anticancer agent.

2. The pharmaceutical composition according to claim 1, wherein the platinum-based drug and/or the taxane-based anticancer agent is one or more selected from the group consisting of cisplatin, carboplatin, oxaliplatin, nedaplatin, docetaxel, and paclitaxel.

3. The pharmaceutical composition according to claim 1, wherein the disease caused by a type-I allergic reaction is one or more diseases selected from the group consisting of fever, nausea, chills, headache, rash, eruption, itching, swelling, tachycardia, hives, flushing, throat tightness, coughing, difficulty breathing, wheezing, loss of consciousness, hypotension, and anaphylaxis accompanied by respiratory problems.

4. The pharmaceutical composition according to claim 1, which is administered in combination with a platinum-based drug and/or a taxane-based anticancer agent.

5. The pharmaceutical composition according to claim 1, which is administered before administration of a platinum-based drug and/or a taxane-based anticancer agent.

6. The pharmaceutical composition according to claim 1, wherein the lecithinized superoxide dismutase is lyophilized.

7. The pharmaceutical composition according to claim 1, comprising a lecithinized superoxide dismutase,
the lecithinized superoxide dismutase comprising a lecithinized superoxide dismutase in which at least one amino group in a human-derived superoxide dismutase subunit coordinated with copper and/or zinc and having a mercapto group in cysteine at position 111 replaced by a hydroxyethylthio group is modified with a lecithin residue of the following formula (I): 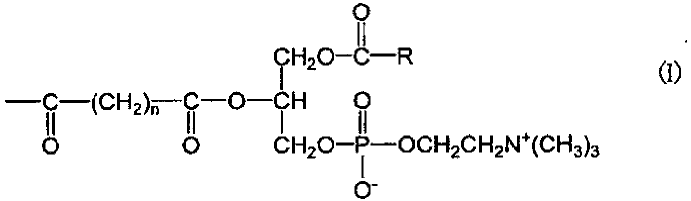 [wherein R represents an alkyl group with 8 to 30 carbon atoms, and n is an integer of from 2 to 10] directly or via a linker, wherein
the lecithinized superoxide dismutase comprises a lecithinized superoxide dismutase (A), as a principal component, constituted by lecithinized superoxide dismutase subunits each of which independently has m number of amino groups [m is an integer of from 1 to 4, and the mean value of m in lecithinized superoxide dismutase subunits is from 1.5 to 2.4] substituted with the lecithin residue, wherein
the lecithinized superoxide dismutase (A) is constituted by the following lecithinized superoxide dismutase subunits:
from 25 to 40 mol% lecithinized superoxide dismutase subunit (a1) with m = 1,
from 35 to 50 mol% lecithinized superoxide dismutase subunit (a2) with m = 2,
from 10 to 20 mol% lecithinized superoxide dismutase subunit (a3) with m = 3, and
from 5 to 15 mol% lecithinized superoxide dismutase subunit (a4) with m = 4.

8. The pharmaceutical composition according to claim 1, wherein PC molecules bind to each SOD molecule with the following binding rates in the lecithinized superoxide dismutase:
(1) the PC binding rate at the binding sites of K3, N terminus, and T2 is from 1% to 10% as the sum of these amino acid residues;
(2) the PC binding rate at the binding site of K9 is from 10% to 30%;
(3) the PC binding rate at the binding site of K23 is from 50% to 75%;
(4) the PC binding rate at the binding site of K36 is from 30% to 55%;
(5) the PC binding rate at the binding site of K70 or K75 is from 10% to 80% as the sum of these amino acid residues;
(6) the PC binding rate at the binding site of K122 or K128 is from 10% to 45% as the sum of these amino acid residues;
(7) the PC binding rate at binding site of K136 is from 10% to 60%; and
on average 1 to 8 PC molecules are bound in a PC-SOD molecule as the sum of the (1) to (7).

9. The pharmaceutical composition according to claim 1, wherein a superoxide dismutase in the lecithinized superoxide dismutase has an amino acid sequence set forth in SEQ ID NO: 1.

10. Use of a lecithinized superoxide dismutase to manufacture a pharmaceutical composition for treating or preventing a disease caused by a type-I allergic reaction caused by administration of a platinum-based drug and/or a taxane-based anticancer agent.

11. A lecithinized superoxide dismutase for treating or preventing a disease caused by a type-I allergic reaction induced by administration of a platinum-based drug and/or a taxane-based anticancer agent.

12. A method for treating or preventing a disease caused by a type-I allergic reaction induced by administration of a platinum-based drug and/or a taxane-based anticancer agent, comprising administration of a lecithinized superoxide dismutase.
